# EUROPÄISCHE PATENTANMELDUNG

(11) **EP 0 962 199 A1**
(43) Veröffentlichungstag der Anmeldung: **08.12.1999**
(21) Anmeldenummer: 98810510.2
(22) Anmeldetag: 04.06.1998
(51) Int. Cl.: A61F 2/36

(54) **Schaftprothese**

(71) Anmelder: Sulzer Orthopädie AG, 6340 Baar (CH)
(72) Erfinder: Puhl, Wolfhart, Prof. Dr. Med., 89081 Ulm (DE); De Cesaris, Alessandro, 8404 Winterthur (CH); Menzi, Manfred, 6340 Baar (CH)
(74) Vertreter: Sulzer Management AG

(57) **Zusammenfassung**

Die Schaftprothese (1) umfasst einen distalen Abschnitt (2) , der einen im wesentlichen kreisförmigen Querschnitt aufweist, und einen sich an diesen distalen Abschnitt (2) anschliessenden proximalen Abschnitt (3), an welchem auf der lateralen Seite eine nach ventral vorstehenden Rippe (4) vorgesehen ist. Der proximale Abschnitt (3) erweitert sich nach oben hin. Im oberen Endbereich ist ein Hals (5) vorgesehen, auf welchem eine Gelenkkugel anbringbar ist. Der Durchmesser der Schaftprothese (1) erweitert sich vom distalen Ende der Schaftprothese (1) ausgehend im wesentlichen allseitig konisch.

## Beschreibung

Die Erfindung betrifft eine Schaftprothese gemäss dem Oberbegriff des unabhängigen Patentanspruchs.

Schaftprothesen, speziell solche zur Implantation im Femur, sind in zahlreichen unterschiedlichen Ausführungen auf dem Markt erhältlich. Hinsichtlich der Art und Weise der Verankerung der Prothese im Femur kann man die Schaftprothesen in zementierte und zementfreie Prothesen unterscheiden. Dabei wird bei der Operationsplanung aufgrund von Informationen, die der Orthopäde von Röntgenbildern etc. über den Knochen erhält, z.B. über die Grösse des Femurs und über dessen Beschaffenheit, festgelegt, ob eine zementierte oder zementfreie Verankerung der Prothese in Frage kommt. Ausserdem wird auch die Grösse der jeweiligen Prothese, die implantiert werden soll, festgelegt.

Bei der Operation wird dann davon ausgegangen, dass eine bestimmte Art der Verankerung erfolgt, z.B. eine zementierte Verankerung. Für eine derartige Verankerung geeignete Prothesen sind beispielsweise bekannt aus der DE-B2-25 37 807 oder aus der EP-A-0,672,396. Zunächst wird bei der Operation von dem Orthopäden das Femur vorbereitet. Vereinfacht ausgedrückt wird dabei eine Höhlung im Femur hergestellt, in welche anschliessend der Zement eingebracht wird. Danach wird die Prothese in den Zement eingeführt und in Position gehalten, bis der Zement hart wird und somit die Prothese fixiert ist. Das Einführen der Prothese in den Zement muss so erfolgen, dass es zu keinen Lufteinschlüssen im Zement kommt. Dieses Einführen erfordert von dem Orthopäden einiges Geschick, da der Zement zum Zeitpunkt des Einbringens in den Femur von seinen Fliesseigenschaften her so beschaffen ist, dass ein Zurückfliessen von einmal verdrängtem Zement praktisch nicht stattfindet. Dies spiegelt sich entsprechend im Schaftdesign wider - das Design von zu zementierenden Prothesen unterscheidet sich im allgemeinen von dem Design von zementfrei zu verankernden Prothesen wesentlich, weil die Verankerung von zementierten Prothesen in dem Zementmantel erfolgt, der die Prothese umgibt, während bei der zementfrei zu verankernden Prothesen die Verankerung direkt in dem die Prothese umgebenden Knochengewebe erfolgt.

Die Entscheidung, ob eine Prothese bei der Implantation zementiert oder zementfrei im Femur verankert wird, wird also bereits vor der Operation, nämlich bei der präoperativen Planung, getroffen. Auch die Entscheidung über die Grösse der zu implantierenden Prothese ist vor der Operation bereits getroffen. Diese Entscheidungen bestimmen dann auch, wie die herzustellende Höhlung zur Aufnahme des Zements von dem Orthopäden aussehen muss bzw. welche Abmessungen sie aufweisen muss.

In einigen Fällen stellt sich jedoch während der Operation heraus, nämlich bei der Vorbereitung des Femurs, dass beispielsweise aufgrund der Beschaffenheit des Knochenmaterials es möglich oder sogar wünschenswert wäre, eine zementfreie Verankerung vorzusehen, obwohl eine zementierte Verankerung geplant ist. Zu diesem Zeitpunkt ist eine Änderung der Entscheidung für die zementierte Prothese jedoch nicht mehr möglich, weil hierzu aufgrund des unterschiedlichen Schaftdesigns von zu zementierenden und von zementfrei zu verankernden Prothesen eine neue Operationsplanung erfolgen müsste.

Es ist daher Aufgabe der Erfindung, eine Schaftprothese vorzuschlagen, die es ermöglicht, eine bereits bei der Operationsplanung erfolgte Entscheidung hinsichtlich der Verankerung noch einmal zu ändern, ohne dass dazu eine neue Planung erforderlich wird. Dies heisst mit anderen Worten, dass die Prothese von ihrem Design her sowohl für eine zementierte als auch für eine zementfreie Verankerung geeignet sein muss.

Erfindungsgemäss wird diese Aufgabe durch eine Schaftprothese gelöst wie sie durch die Merkmale des unabhängigen Patentanspruchs charakterisiert ist. Insbesondere weist die erfindungsgemässe Schaftprothese einen distalen Abschnitt auf, der einen im wesentlichen kreisförmigen Querschnitt aufweist. Das heisst, dass dieser distale Abschnitt vorzugsweise komplett kreisförmig ausgebildet ist, jedoch geringe Abweichungen von der Kreisform möglich sind, wie z.B. Abflachungen, um bei zementierten Verankerungen einen genügend dicken Zementmantel zu gewährleisten. An diesen distalen Abschnitt schliesst sich ein proximaler Abschnitt an, an welchem auf der lateralen Seite eine nach ventral vorstehenden Rippe vorgesehen ist. Der proximalen Abschnitt der Schaftprothese erweitert sich nach oben hin und im oberen Endbereich ist ein Hals vorgesehen, auf welchem eine Gelenkkugel anbringbar ist. Der Durchmesser der Schaftprothese erweitert sich vom distalen Ende der Schaftprothese ausgehend im wesentlichen allseitig konisch (siehe oben), vorzugsweise allseitig konisch. Eine Schaftprothese dieser Art eignet sich grundsätzlich sowohl für eine zementierte als auch für eine zementfreie Verankerung im Knochen.

Die vom distalen Ende der Schaftporthese ausgehende im wesentlichen allseitige konische Erweiterung bedeutet, dass die Prothese keine Hinterschneidungen aufweist, welche sonst bei einer zementierten Verankerung wegen der schlechten Fliesseigenschaften (hohe Viskosität) des Knochenzements zu Lufteinschlüssen führen können. Zudem führt die Erweiterung des Schafts in proximaler Richtung zu einer Kompression des Knochenzements und dadurch zu besserer Verteilung des Zements in Hohlräume in den oft porösen Knochen (Spongiosa) hinein. Darüberhinaus entspricht eine bereits vom distalen Ende der Prothese ausgehende konische Erweiterung dem natürlichen Verlauf des Markraums im diaphysären Knochenbereich. Bei Schaftprothesen mit nicht-konischen zylindrischen distalen Endbereichen kann es nämlich bei der zementfreien Verankerung teilweise zu Verklemmungen solcher Prothesen kommen, deren Verankerung jedoch vorzugsweise im proximalen Bereich der Prothese erfolgen soll. Eine solche Verklemmung bedeutet dann für den Orthopäden einen zusätzlichen Arbeitsschritt, da er den mit einer konischen Reibahle vorbereiteten Markraum dann noch zusätzlich aufbohren muss, damit die Prothese noch weiter in den Markraum eingeführt werden kann, damit sie im proximalen Endbereich verankert werden kann.

Bei einem vorteilhaften Ausführungsbeispiel der erfindungsgemässen Schaftprothese liegt der Öffnungswinkel im Bereich des sich konisch erweiternden distalen Abschitts im Bereich von 1° bis 10°, insbesondere beträgt er etwa 2°. Mit diesem Bereich sind praktisch alle vorkommenden Verläufe des Markraums im diaphysären Knochenbereich erfasst, ein nicht selten vorkommender Verlauf weist einen Öffnungswinkel von 2° auf.

Bei einem weiteren vorteilhaften Ausführungsbeispiel der erfindungsgemässen Schaftprothese liegt der Winkel zwischen der Schaftlängsachse und der Halsachse im Bereich von 120° bis 150°, insbesondere beträgt er etwa 135°. Bei diesem Winkel spricht man auch von dem sogenannten "CCD-Winkel" (Centrum-Collum-Diaphyse-Winkel). Mit dem genannten Bereich sind ebenfalls die wesentlichen in der Praxis auftretenden Fälle erfasst. Ein CCD-Winkel von 135° wird häufig bei jüngeren Patienten angetroffen, jedoch ist eine Prothese mit einem CCD-Winkel dieser Grösse durchaus auch für Patienten fortgeschritteneren Alters implantierbar. Mit einem solchen CCD-Winkel kann dann z.B. eine Straffung des teilweise erschlafften Bandapparats bewirkt werden, weil der natürliche CCD-Winkel am Knochen des Patienten vor der Implantation der Prothese geringer war und durch den grösseren CCD-Winkel der Bandapparat wieder "gespannt" wird.

Bei einem weiteren Ausführungsbeispiel der erfindungsgemässen Schaftprothese ist die Schaftprothese im proximalen Abschnitt in Richtung nach anterior geneigt ist, wobei dieser Neigungswinkel oder Anteversionswinkel (Anteversionswinkel ist die häufig angetroffene Bezeichnung) im Bereich von 5° bis 17° liegt, insbesondere etwa 11° beträgt. Dieser Winkelbereich umfasst die am häufigsten vorkommenden Anteversionswinkel, ein sehr häufig auftretender Wert für diesen Winkel beträgt etwa 11°.

Ein weiterer Aspekt der vorliegenden Erfindung betrifft eine Reihe von Schaftprothesen unterschiedlicher Grössen, wobei die einzelnen Schaftprothesen einer solchen Reihe wie vorstehend beschrieben ausgebildet sind. Die Schaftprothesen unterschiedlicher Grössen sind nach dem Zwiebelschalenprinzip aufgebaut, wobei die Dickenabmessungen von zwei aufeinanderfolgenden Grössen sich um einen konstanten Betrag unterscheiden. Die Länge von zwei aufeinanderfolgenden Grössen kann sich ebenfalls um einen konstanten Betrag unterscheiden, der aber von dem Betrag des Dickenunterschiedes abweichen kann.

Bei der zementierten Verankerung existieren verschiedene Philosophien hinsichlich der Dicke des Zementmantels, der die implantierte Prothese umgibt, nämlich solche Philosophien, die von einem sehr dünnen Zementmantel ausgehen (z.B. wenige Zehntel Millimeter) und solche, die von einem dickeren Zementmantel ausgehen (z.B. im Bereich von einem bis hin zu drei Millimetern). Hierbei ist unter dem Zementmantel der mit Zement gefüllte Zwischenraum zwischen der vorbereiteten Implantathöhlung und der Prothese zu verstehen. Es ist klar, dass ein Eindringen von Zement in die Spongiosa in der Praxis zu einer Verdickung dieses Zementmantels führen kann. Insbesondere im Hinblick auf die letztere Philosophie (dickerer .Zementmantel) betrifft ein weiterer Aspekt der vorliegenden Erfindung einen Satz von Schaftprothesen, welcher zwei Reihen von Schaftprothesen - eine erste und eine zweite Reihe - umfasst, wobei die Prothesen einer Reihe so ausgebildet sind wie vorstehend beschrieben. In der ersten Reihe sind Schaftprothesen für zementfreie Implantationen enthalten und in der zweiten Reihe Schaftprothesen solche für zementierte Implantationen sind. Die Schaftprothesen der verschiedenen Reihen sind dabei von ihrem Design und von ihrer Grösse her mit der entsprechenden Schaftprothese aus der anderen Reihe identisch, jedoch ist diejenige Schaftprothese aus der zweiten Reihe (Prothesen für zementierte Verankerung), welche einer Schaftprothese für zementfreie Implantation aus der ersten Reihe entspricht, kleiner als die entsprechende Schaftprothese in der ersten Reihe. Lediglich der Hals der Schaftprothese aus der zweiten Reihe (Prothesen für zementierte Verankerung) entspricht in etwa der Grösse des Halses der entsprechenden Prothese aus der ersten Reihe (zementfreie Verankerung), wodurch vom Hals der Prothese her die Grösse in etwa gleich ist wie bei der zementfreien Verankerung, die zu zementierende Prothese selbst jedoch kleiner ist als die entsprechende Prothese für eine zementfreie Verankerung.

Weitere vorteilhafte Ausgestaltungen der Erfindung ergeben sich aus den nachfolgend anhand der Zeichnung beschriebenen Ausführungsbeispielen. Es zeigen, teilweise schematisch und/oder im Schnitt:
- Fig. 1: ein Ausführungsbeispiel einer erfindungsgemässen Schaftprothese in perspektivischer Darstellung,
- Fig. 2: das Ausführungsbeispiel der Schaftprothese aus Fig. 1 in einer Ansicht von ventral,
- Fig. 3: das Ausführungsbeispiel der Schaftprothese aus Fig. 1 in einer Ansicht von lateral nach medial
und
- Fig. 4-8: Schnitte durch das Ausführungsbeispiel der Schaftprothese aus Fig. 2 entlang den Linien IV-IV, V-V, VI-VI, VII-VII und VIII-VIII.

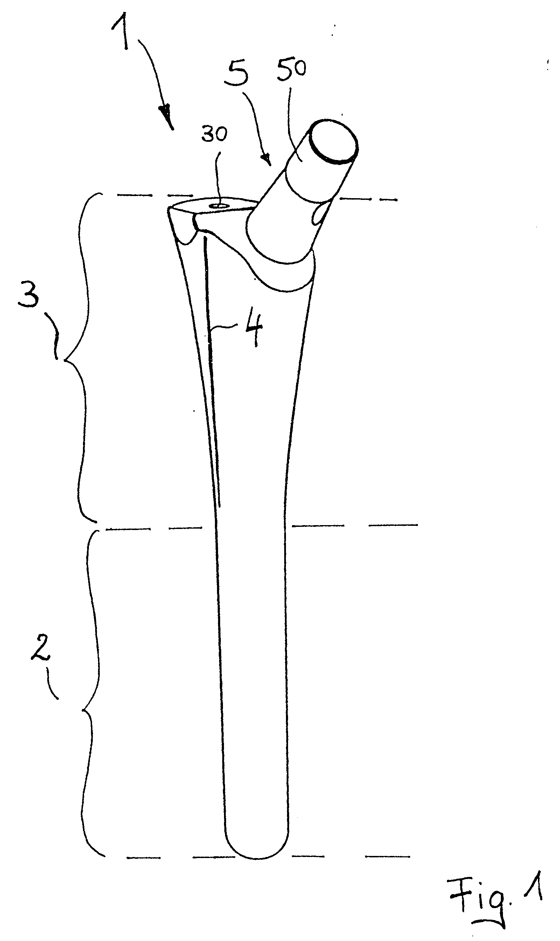

In Fig. 1 ist ein Ausführungsbeispiel einer erfindungsgemässen Schaftprothese in einer perspektivischen Ansicht aus medialer/ventraler Richtung gezeigt. Die Schaftprothese 1 umfasst einen distalen Abschnitt 2 und einen sich an diesen distalen Abschnitt anschliessenden proximalen Abschnitt 3. Am oberen Ende des proximalen Abschnitts 3 ist eine Vertiefung 30 zum Ansetzen eines Einschlaginstruments für zementfreie Verankerung bzw zum Ansetzen eines Eindrückinstruments für zementierte Verankerung zu erkennen. Auf der lateralen Seite steht am proximalen Abschnitt 3 eine Rippe 4 in ventraler Richtung vo. Diese Rippe 4 stellt in etwa die anatomischen Gestalt des Knochens im lateralen Bereich nach. Im oberen Endbereich des proximalen Abschnitts 3 der Schaftprothese 1 schliesst sich ein Hals 5 an, auf welchem eine Gelenkkugel (nicht dargestellt) anbringbar ist. Dies erfolgt in dem gezeigten Ausführungsbeispiel derart, dass auf das konisch zulaufende Halsstück 50 eine mit einer konischen Sacklochbohrung ausgebildete Gelenkkugel aus einem geeigneten Material (z.B. aus Keramik oder aus Metall) aufgesteckt und über den Konusklemmsitz auf dem Halsstück 50 gehalten wird.

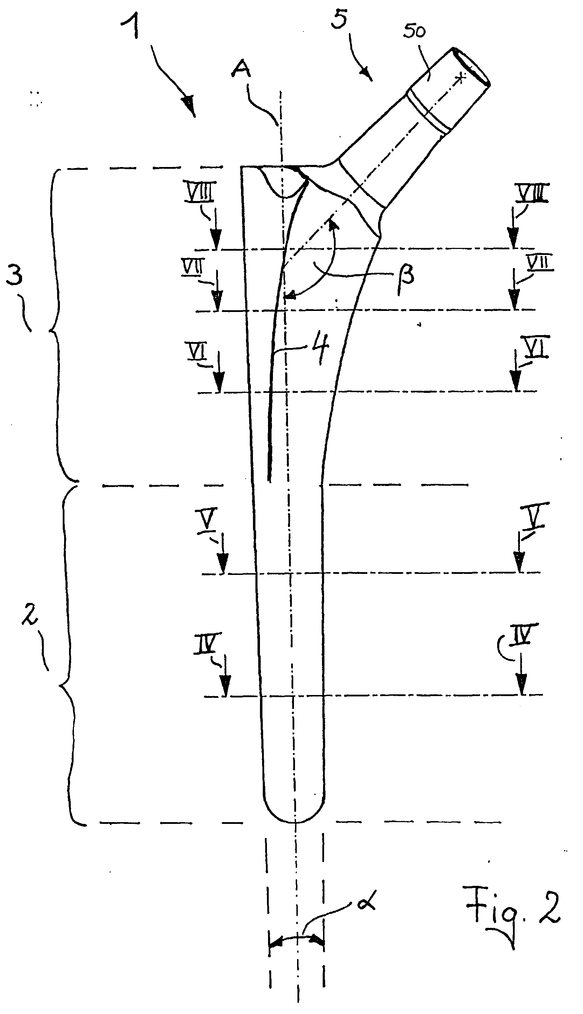

In Fig. 2 ist das Ausführungsbeispiel der Schaftprothese 1 aus Fig. 1 in einer Ansicht von ventral zu erkennen. Man erkennt auch hier den distalen Abschnitt 2 und den sich daran anschliessenden proximalen Abschnitt 3 der Schaftprothese 1. Ferner erkennt man die nach ventral (also aus der Zeichenebene quasi hervorstehende) Rippe 4 und den im oberen Endbereich des proximalen Abschnitts sich anschliessenden Hals 5. Ferner erkennt man in Fig. 2, dass sich der Durchmesser der Schaftprothese vom distalen Ende der Schaftprothese ausgehend allseitig erweitert. Dies ist in Fig. 2 mit Hilfe des Öffnungswinkels α angedeutet, welcher im Bereich von 1° bis 10° liegt. Insbesondere beträgt er etwa 2°. Man muss sich den Öffnungswinkel α in Fig. 2 so vorstellen, dass sowohl die beiden gestrichelten Linien, die am distalen Ende der Prothese dargestellt sind (quasi als fiktive Verlängerung der Aussenkontur der Prothese), als auch die strichpunktiert dargestellte Achse A so lange weitergezogen werden, bis sich diese drei Linien schneiden (was weit unterhalb des Papiers erst der Fall wäre). Der Öffnungswinkel α wird dann von den beiden gestrichelten Linien eingeschlossen.

Ferner erkennt man in Fig. 2 noch den Winkel β, den die Achse des Halses 5 mit der Schaftachse 10 einschliesst. Dieser Winkel wird auch CCD-Winkel (Centrum-Collum-Diaphyse-Winkel) genannt. Dieser CCD-Winkel β liegt bei dem gezeigten Ausführungsbeispiel der Schaftprothese im Bereich von 120° bis 150°, insbesondere beträgt er etwa 135°. Ein CCD-Winkel von 135° ist im Femur häufiger bei jüngeren Patienten anzutreffen, bei älteren Patienten ist er in der Regel kleiner. Dennoch ist eine Prothese mit einem solchen CCD-Winkel durchaus auch bei Patienten fortgeschritteneren Alters implantierbar. Eine Implantation einer Prothese mit einem solchen CCD-Winkel kann dann bei solchen Patienten eine Straffung des teilweise erschlafften Bandapparats zur Folge haben, der Bandapparat wird also durch den grösseren CCD-Winkel quasi wieder "gespannt" und aktiviert.

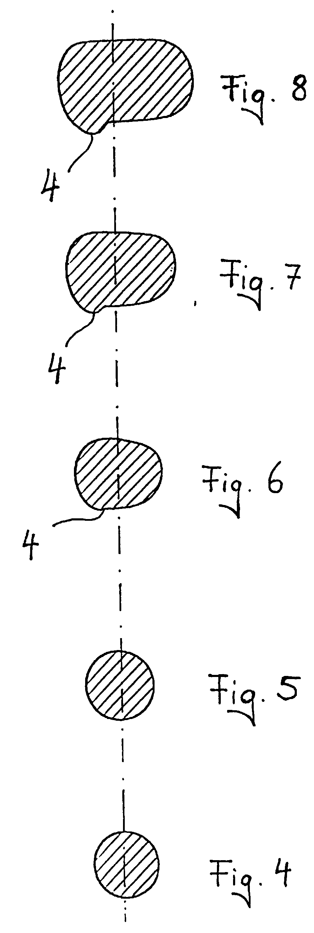

In Fig. 2 erkennt man darüberhinaus noch die verschiedenen Schnitte IV-IV, V-V, VI-VI, VII-VII und VIII-VIII. Die Schnitte selbst sind in Fig. 4-8 dargestellt. In Fig. 4 erkennt man einen Querschnitt im distalen Abschnitt 2 der Schaftprothese 1. Der Querschnitt ist kreisförmig, was auch noch für den in Fig. 5 gezeigten Querschnitt V-V im distalen Abschnitt 2 gilt. Dieser Querschnitt ist jedoch vom Durchmesser her grösser als der Querschnitt IV-IV, weil sich ja die Schaftprothese vom distalen Ende ausgehend kontinuierlich allseitig erweitert. Der in Fig. 6 gezeigte Querschnitt VI-VI befindet sich bereits in dem proximalen Abschnitt 3 der Prothese. Dort erkennt man bereits schwach die auf der lateralen Seite der Schaftprothese nach ventral vorstehende Rippe 4. Ausserdem erkennt man, dass sich die Schaftprothese im proximalen Abschnitt 3 allseitig noch erweitert. Dies erkennt man speziell auch in Fig. 2.

In Fig. 7 ist der Querschnitt entlang der Linie VII-VII der Fig. 2 zu erkennen, bei welchem die auf der lateralen Seite der Schaftprothese 1 nach ventral vorstehende Rippe 4 noch stärker ausgeprägt zu erkennen ist als im Querschnitt entlang der Linie VI-VI. Am stärksten ausgeprägt ist diese nach ventral vorstehende Rippe 4 in Fig. 8 zu erkennen, welche einen Querschnitt entlang der Linie VIII-VIII der Fig. 2 zeigt.

Insgesamt erkennt man aus den Fig. 4-8, dass sich die Prothese 1 direkt vom distalen Ende her beginnend im distalen Abschnitt 2 allseitig konisch erweitert, und dass sich die Erweiterung der Prothese im proximalen Abschnitt 3 fortsetzt. Diese Gestaltung der Prothese hat zum einen den Vorteil, dass keine Hinterschneidungen existieren, sodass bei zementierten Verankerungen keine Lufteinschlüsse zu befürchten sind, die im Falle von Hinterschneidungen auftreten könnten, weil der Knochenzement beim Einführen der Prothese in den Zement nur noch schlecht fliessfähig (also hochviskos) ist, und ein Zurückfliessen von bereits von der Prothese verdrängtem Zement quasi nicht erfolgt. Zum anderen hat diese Gestaltung der Prothese den Vorteil, dass die vom distalen Ende ausgehende allseitig konische Erweiterung der Prothese im distalen Abschnitt 2 der Prothese insbesondere auch bei zementfreien Verankerungen das Einführen der Prothese in den Markraum im diaphysären Bereich des Femurs erleichtert, weil der Markraum im diaphysären Bereich des Femurs ebenfalls leicht konisch verlaufend ausgebildet ist. Bei nicht-konisch zylindrischen distalen Abschnitten von Prothesen kann es hingegen zu Verklemmungen beim Einführen der Prothese kommen, sodass der Orthopäde dann den Markraum zusätzlich aufbohren muss, um die bei zementfreien Verankerungen gewünschte proximale Verankerung zu bewirken. Dies würde aber einen zusätzlichen Arbeitsschritt für den Orthopäden bei der Präparation des Femurs bedeuten. Bei zementierten Verankerungen wird durch die allseitig konische Erweiterung im distalen Abschnitt 2 der Prothese und die anschliessende Erweiterung im proximalen Bereich 3 der Prothese eine Kompression auf den Zement bewirkt und dadurch zu besserer Verteilung des Zements in Hohlräume in der Spongiosa.

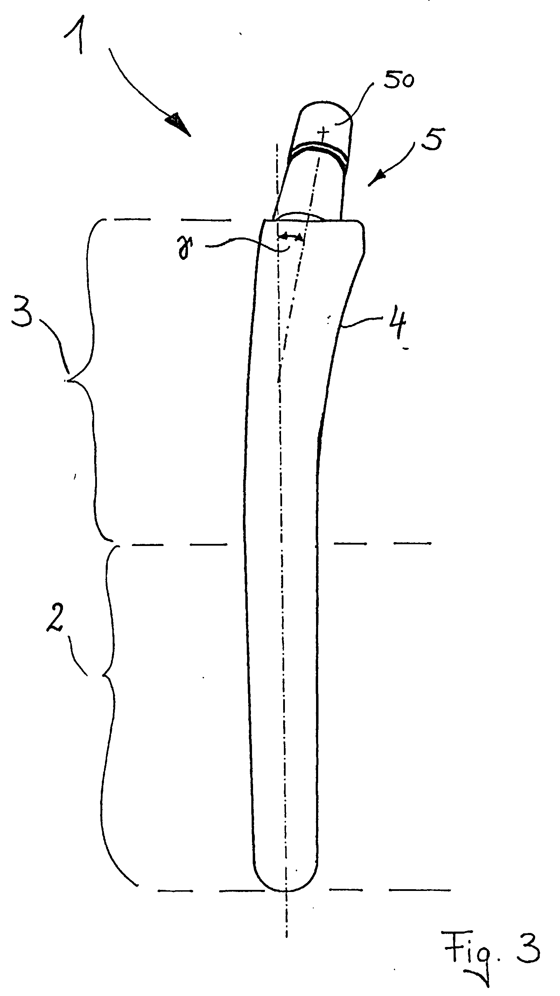

Fig. 3 zeigt schliesslich noch das Ausführungsbeispiel der Schaftprothese 1 aus Fig. 1 in einer Ansicht von lateral nach medial. Man erkennt in dieser Ansicht ebenfalls die nach ventral vorstehende Rippe 4. Ferner erkennt man noch, dass die Prothese im proximalen Abschnitt in Richtung nach anterior geneigt ist. Dieser Neigungswinkel γ, der häufig auch als Anteversionswinkel bezeichnet wird, liegt im Bereich von 5° bis 17°, insbesondere beträgt er etwa 11°. Dieser Bereich umfasst die am häufigsten auftretenden Werte für die Anteversionswinkel, der Wert von etwa 11° für den Anteversionswinkel γ entspricht einem der am häufigsten anzutreffenden Werte.

Da die vorkommenden Femurgrössen sich durchaus stark unterscheiden können, muss eine ganze Reihe von Prothesen bereitgestellt werden, um das gesamte Spektrum von Femurgrössen abdecken zu können. Bei der präoperativen Planung wird zwar eine ganz bestimmte Grösse einer Prothese für den Patienten ermittelt, jedoch kann es sich bei der Operation schliesslich herausstellen, dass entweder das nächstgrössere Implantat oder das nächstkleinere Implantat unter Umständen besser geeignet wäre. Aus diesem Grunde betrifft ein weiterer Aspekt der Erfindung eine Reihe von Schaftprothesen unterschiedlicher Grösse. Die einzelnen Schaftprothesen einer solchen Reihe sind so ausgebildet wie vorstehend beschrieben. Die Schaftprothesen einer solchen Reihe sind nach dem sogenannten "Zwiebelschalenprinzip" aufgebaut, das heisst, dass sich die Dickenabmessungen von zwei aufeinanderfolgenden Grössen von Schaftprothesen um einen konstanten Betrag unterscheiden. Auch die Längenabmessungen von zwei aufeinanderfolgenden Grössen von Schaftprothesen können sich um einen konstanten Betrag unterscheiden, der aber wiederum verschieden von dem Unterschied in den Dickenabmessungen sein kann.

Insbesondere beim Verankern der Prothese mittels Knochenzement existieren unterschiedliche Philosophien. Diese unterscheiden sich wesentlich durch die Dicke des Zementmantels, der die Prothese umgibt (also die Dicke der Zementschicht zwischen Höhlung im Femur und der Prothese). Manche Philosophien gehen von sehr dünnen Zementmänteln aus, die im Bereich von einigen Zehntel Millimetern liegen, andere gehen von Zementmänteln aus, die im Bereich von etwa eins bis drei Millimetern liegen. Insbesondere bei Zementmänteln, die im letztgenannten Bereich liegen, ist es allerdings in Bezug auf die Prothesengrösse sehr erheblich, ob nun eine zementierte oder eine zementfreie Verankerung erfolgt. War beispielsweise eine zementfreie Verankerung präoperativ geplant und hat sich bei der Operation herausgestellt, dass eine zementierte Verankerung angezeigt ist, so muss eine von den Abmessungen her deutlich kleinere Prothese gewählt werden. Der Hals der kleineren Prothese für die zementierte Verankerung muss jedoch im wesentlichen die gleiche Grösse aufweisen wie der Hals der Prothese für die zementfreie Verankerung. Daher betrifft ein weiterer Aspekt der Erfindung einen Satz von Schaftprothesen, wobei dieser Satz von Schaftprothesen zwei Reihen von Prothesen umfasst, die wie vorstehend beschrieben ausgebildet sind. In einer ersten Reihe sind Schaftprothesen für eine zementfreie Verankerung enthalten, während in einer zweiten Reihe Schaftprothesen für zementierte Verankerungen enthalten sind. Dabei kann entweder für jede einzelne zementfrei zu verankernde Prothese (aus der ersten Reihe) eine entsprechende zu zementierende Prothese (in der zweiten Reihe) im Satz enthalten sein, es kann aber auch so sein, dass z.B. für zwei benachbarte Grössen von zementfrei zu verankernden Prothesen nur eine Prothese mit einer Zwischengrösse für die zementierte Verankerung (in der zweiten Reihe) im Satz enthalten ist. Diejenige Schaftprothese aus der zweiten Reihe (zementierte Verankerung), die einer Schaftprothese für eine zementfreie Verankerung aus der ersten Reihe entspricht, ist kleiner als die entsprechende Schaftprothese aus der ersten Reihe. Die Grösse des Halses einer solchen Prothese aus der zweiten Reihe (zementierte Verankerung) entspricht jedoch in etwa der Grösse des Halses der entsprechenden Prothese für die zementfreie Verankerung, was auch bei Zementmänteln der angesprochenen Dicke noch eine intraoperative Entscheidung darüber ermöglicht, ob nun eine zementfreie oder zementierte Verankerung der Prothese erfolgen soll.

Es ist selbstverständlich, dass die Materialien für eine zementfrei zu verankernde Schaftprothese und für eine zu zementierende Prothese unterschiedlich sind. Während für zementfrei zu verankernde Prothesen vorzugsweise Titanlegierungen zur Anwendung kommen wegen der Biokompatibilität solcher Legierungen (schliesslich muss ja der Knochen auf die Prothese aufwachsen), kommen für zu zementierende Prothese beispielsweise Kobalt-Chrom-Molybdän-Legierungen in Betracht (CoCrMo-Legierungen). Gleichwohl können die Prothesen für zementfreie Verankerung und für zementierte Verankerung vom Design her durchaus gleich sein, da das Design der Schaftprothesen für beide Verankerungsarten geeignet ist.

## Patentansprüche

1. Schaftprothese (1) mit einem distalen Abschnitt (2), der einen im wesentlichen kreisförmigen Querschnitt aufweist, und mit einem sich an diesen distalen Abschnitt (2) anschliessenden proximalen Abschnitt (3), an welchem auf der lateralen Seite eine nach ventral vorstehenden Rippe (4) vorgesehen ist, bei welcher Schaftprothese (1) sich ferner der proximale Abschnitt (3) nach oben hin erweitert und bei welcher im oberen Endbereich ein Hals (5) vorgesehen ist, auf welchem eine Gelenkkugel anbringbar ist, dadurch gekennzeichnet, dass sich der Durchmesser der Schaftprothese (1) vom distalen Ende der Schaftprothese (1) ausgehend im wesentlichen allseitig konisch erweitert.

2. Schaftprothese nach Anspruch 1, dadurch gekennzeichnet, dass der Öffnungswinkel (α) im Bereich des sich konisch erweiternden distalen Abschitts im Bereich von 1° bis 10° liegt, insbesondere etwa 2° beträgt.

3. Schaftprothese nach Anspruch 1 oder 2, dadurch gekennzeichnet, dass der Winkel (β) zwischen der Schaftlängsachse (A) und der Halsachse im Bereich von 120° bis 150° liegt, insbesondere etwa 135° beträgt.

4. Schaftprothese nach einem der vorangehenden Ansprüche, dadurch gekennzeichnet, dass die Schaftprothese (1) im proximalen Abschnitt (3) in Richtung nach anterior geneigt ist, wobei dieser Neigungswinkel (γ) oder Anteversionswinkel im Bereich von 5° bis 17° liegt, insbesondere etwa 11° beträgt.

5. Reihe von Schaftprothesen unterschiedlicher Grössen, wobei die einzelnen Schaftprothesen (1) der Reihe entsprechend einem der vorangehenden Ansprüche ausgebildet sind und die Schaftprothesen unterschiedlicher Grössen nach dem Zwiebelschalenprinzip aufgebaut sind, wobei die Dickenabmessungen von zwei aufeinanderfolgenden Grössen von Schaftprothesen sich um einen konstanten Betrag unterscheiden.

6. Satz von Schaftprothesen umfassend zwei Reihen - eine erste und eine zweite Reihe - von Schaftprothesen (1) gemäss Anspruch 5, wobei in der ersten Reihe Schaftprothesen (1) für zementfreie Implantationen und in der zweiten Reihe Schaftprothesen (1) für zementierte Implantationen enthalten sind, wobei diejenige Schaftprothese aus der zweiten Reihe, die einer Schaftprothese für zementfreie Implantation aus der ersten Reihe entspricht, kleiner ist als die entsprechende Schaftprothese in der ersten Reihe, jedoch der Hals (5) der entsprechenden Schaftprothese (1) aus der zweiten Reihe in etwa der Grösse des Halses (5) der entsprechenden Schaftprothese (1) aus der ersten Reihe entspricht.
